# EUROPEAN PATENT APPLICATION

(11) **EP 1 764 029 A1**
(43) Date of publication of application: **21.03.2007**
(21) Application number: 05765469.1
(22) Date of filing: 05.07.2005
(51) Int. Cl.: A61B 1/00

(54) **ENDOSCOPE**

(30) Priority: 07.07.2004 JP 2004201088
(71) Applicant: Olympus Corporation, Tokyo 151-0072 (JP)
(72) Inventor: IIJIMA, Kazuo, 1920046 (JP)
(74) Representative: von Hellfeld, Axel
(86) International application number: PCT/JP2005/012418
(87) International publication number: WO 2006/004124

(57) **Abstract**

Provided is an endoscope which has favorable observability and an illuminance distribution shows no decrease in illuminance at a central portion of an observed area even when the area is observed at a relatively short distance with the use of an endoscope having a wide viewing angle. The endoscope has an insertion portion to be inserted into a subject body, and includes an observation optical member which is provided at a distal end portion of the insertion portion and serves for an observation of the subject body, at least two first illumination members each of which is arranged around the observation optical member and has an axis defined by a center of an illumination range forming a predetermined angle with an optical axis of the observation optical member in a radially outward direction of the insertion portion, and a second illumination member which is arranged around the observation optical member. An angle formed by an axis defined by a center of an illumination range of the second illumination member and the optical axis of the observation optical member is smaller than the predetermined angle formed by an axis defined by a center of an illumination range of the first illumination member and the optical axis of the observation optical member.

## Description

### TECHNICAL FIELD

The present invention relates to an endoscope, and more particularly to an endoscope having a characteristic structure of a distal end portion of an insertion portion.

### BACKGROUND ART

Conventionally, endoscopes have been widely used in a field of medicine or the like. The endoscope has an elongated insertion portion which is inserted into a body cavity. The operator can observe an internal organ or the like inside the body cavity and perform various types of treatment using a treatment instrument inserted into a treatment instrument insertion channel if necessary. At a distal end of the insertion portion, a bendable portion is provided. The operator can change an observation direction of an observation window provided at a distal end portion by operating an operation portion of the endoscope.

A viewing angle of a conventional endoscope is 140°, for example. The operator observes an interior of the body cavity by looking at an observation image corresponding to the viewing angle. When the operator desires to observe a portion outside a viewing range, the operator bend the bendable portion to shift the viewing range thereby observing the portion previously outside the viewing range.

When the operator observe the interior of the body cavity using the above mentioned endoscope, sometimes the operator needs to observe a wall of the body cavity at a relatively short distance. During such a short-distance observation, an illuminance distribution of illuminating light shows a decrease of illuminance at a central portion of an observed area. In view of the above, some propose an endoscope in which a third illuminating unit is newly provided between two illuminating units which are conventionally employed (see Patent Document 1)

Patent Document 1: Japanese Patent Application Laid-Open No. H11-342105

### DISCLOSURE OF INVENTION

### PROBLEM TO BE SOLVED BY THE INVENTION

However, when the operator tries to observe a certain area at a relatively short distance using an endoscope having a wider viewing angle for a wider-range observation, inconvenience caused by the decrease of illuminance in the illuminance distribution at the central portion of the observed area as mentioned above, i.e., deficiency of light intensity at the central portion becomes even more problematic.

In the above mentioned document, however, there is no description nor suggestion on how to deal with the problem as mentioned above when the operator wants to observe a certain area at a relatively short distance using an endoscope with a wider viewing angle.

In view of the above, an object of the present invention is to provide an endoscope with high observability, according to which an illuminance distribution does not show a decrease in illuminance at the central portion of the observed area even when the employed endoscope has a wide viewing angle and the area is observed at a relatively short distance.

### MEANS FOR SOLVING PROBLEM

To solve the problems as described above and to achieve the object, an endoscope according to the present invention has an insertion portion to be inserted into a subject body, and includes an observation optical member which is provided in a distal end portion of the insertion portion and serves for an observation of the subject body; at least two first illumination members each of which is arranged around the observation optical member and having an axis defined by a center of an illumination range forming a predetermined angle with an optical axis of the observation optical member in a radially outward direction of the insertion portion; and a second illumination member which is arranged around the observation optical member, wherein an angle formed by an axis defined by a center of an illumination range of the second illumination member and the optical axis of the observation optical member is smaller than the predetermined angle formed by the axis defined by the center of the illumination range of the first illumination member and the optical axis of the observation optical member.

### EFFECT OF THE INVENTION

According to the present invention, an endoscope with high observability can be realized, according to which an illuminance distribution does not show a decrease in illuminance at the central portion of the observed area even when the employed endoscope has a wide viewing angle and the area is observed at a relatively short distance.

### BRIEF DESCRIPTION OF DRAWINGS

FIG. 1 is a schematic diagram of an endoscope apparatus according to an embodiment of the present invention;
FIG. 2 is an elevation view of a cylindrical distal end portion at a side of a distal end thereof according to the embodiment of the present invention;
FIG. 3 is a sectional view of a distal end portion 10 along line P-P of FIG. 2;
FIG. 4 is a diagram illustrating a relation among an observed area whose image is captured via a lens for an observation window; and
FIG. 5 is a sectional view of a distal end portion according to a modification of the embodiment of the present invention.

### EXPLANATIONS OF LETTERS OR NUMERALS

- 1: Endoscope
- 2: Operation portion
- 3: Insertion portion
- 7: Monitor
- 10: Distal end portion
- 22: Observation window
- 23: Illumination window
- 22c: Imaging element

### BEST MODE(S) FOR CARRYING OUT THE INVENTION

Exemplary embodiments of the present invention will be described with reference to the accompanying drawings. It should be noted that the present invention is not limited to the embodiments.

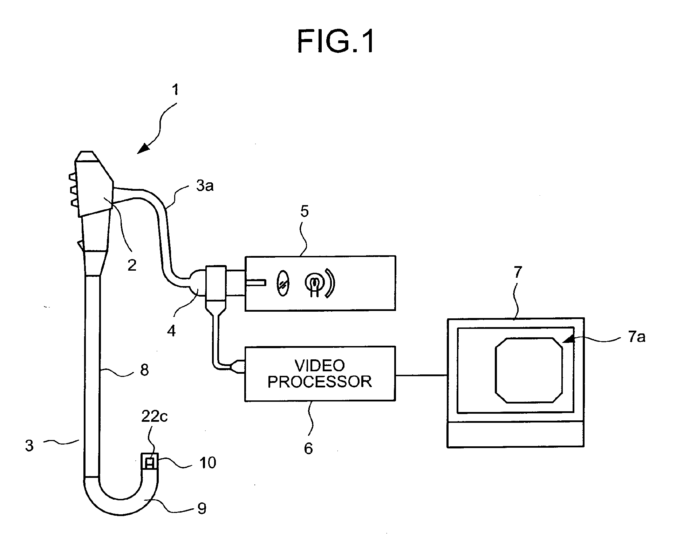

First, a structure of an endoscope apparatus according to the embodiment will be described with reference to FIG. 1. FIG. 1 is a schematic diagram of the endoscope apparatus according to the embodiment of the present invention. As shown in FIG. 1, an endoscope 1 includes an operation portion 2 which controls a bending operation and a piping system, an insertion portion 3 which is connected to the operation portion 2 at a proximal end side and inserted into a body cavity, and a universal chord 3a which extends from the operation portion 2 and has a connector portion 4 at a distal end. The connector portion 4 can be connected to a light source 5 and a video processor 6 via a predetermined connector. The video processor 6 is connected to a monitor 7. The insertion portion 3 has a tube 8 which has flexibility, a bendable portion 9 which is provided at a distal end side of the tube 8, and a distal end portion 10 which is provided at a distal end side of the bendable portion 9. In the distal end portion 10, an imaging element 22c for imaging an area inside the body cavity is incorporated.

The imaging element 22c provided in the distal end portion 10 captures an image of an area inside the body cavity, and image signals corresponding to the image are transmitted to the video processor 6 via the universal chord 3a. The video processor 6 has a signal processing circuit (not shown) which processes the transmitted image signals, and an observation image of the area whose image is captured is displayed based on the processed signals on a display screen 7a of the monitor which is a display unit connected to the video processor 6.

An operation knob is arranged in the operation portion 2 to remotely control the bending of the bendable portion 9. When the operation knob is manipulated, an operation wire (not shown) penetrating the insertion portion 3 is pulled or loosened, which causes the bendable portion 9 to be bent in four directions.

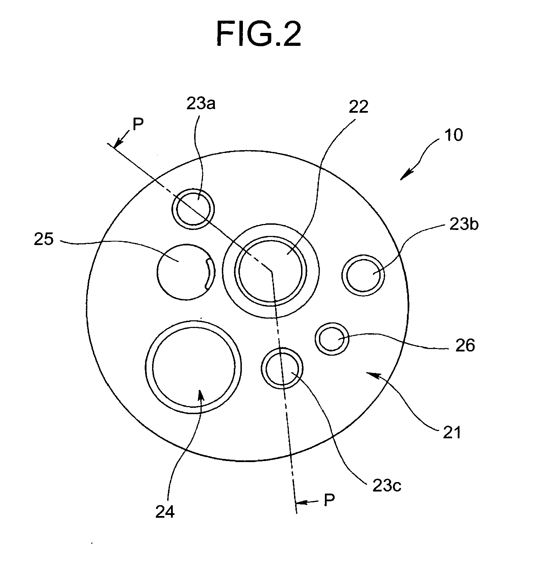

FIG. 2 is an elevational view of the cylindrical distal end portion 10 from the distal end side. On a distal end surface 21 of the distal end portion 10, an observation optical member 22, three illumination members 23a, 23b, and 23c, a treatment instrument insertion channel opening 24, a water delivery nozzle 25 for washing, and a forward water delivery nozzle 26 which serves for washing off blood, mucus, or the like on an affected area of the subject or the like are arranged. Therefore, plural openings are provided on the distal end surface 21 of the distal end portion 10 so that the observation optical member 22, three illumination members 23a, 23b, and 23c (hereinafter three members may be collectively denoted by reference character 23), the treatment instrument insertion channel opening 24, the water delivery nozzle 25, and the forward water delivery nozzle 26.

As shown in FIG. 2, on the distal end surface 21 of the distal end portion 10, three illumination members 23 are arranged around a center of an optical axis of the observation optical member 22 so that the illuminance is uniform within the observed area. Hence, the illuminance within the observed area can be well distributed even though there are a few illumination units, and at the same time the insertion portion 3 can be thinned down. The arrangement of these elements will be described later in detail.

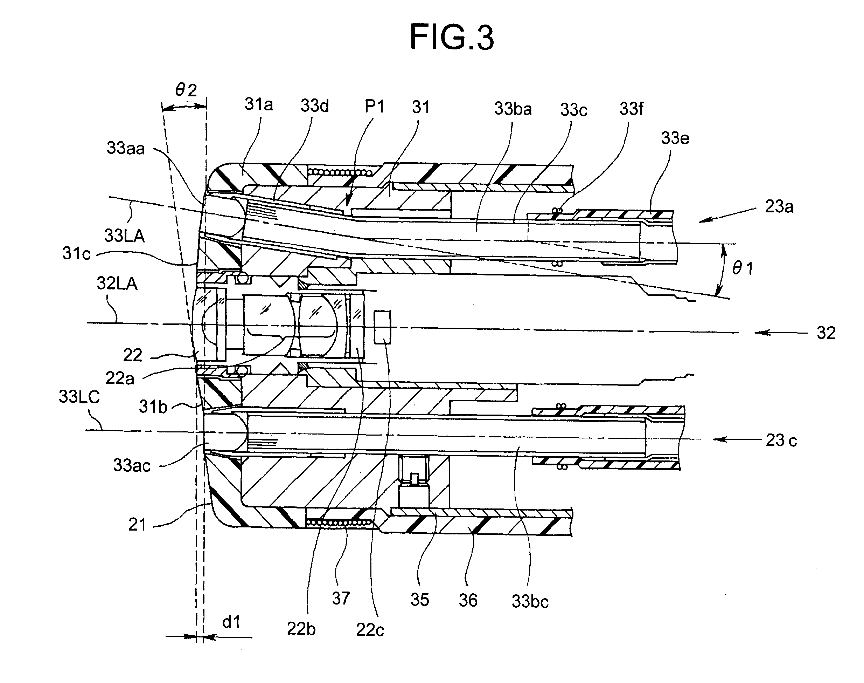

FIG. 3 is a sectional view of the distal end portion 10 along line P-P of FIG. 2. In the distal end portion 10, an imaging unit or the like 32 corresponding to the observation optical member 22 and a distal end hard portion 31 which has a space inside so that a light guide or the like corresponding to the three illumination members 23 can be arranged inside the distal end portion 10 are arranged. Further, a cover 31a is placed so as to cover the distal end side of the distal end hard portion 31. The imaging unit 32 is inserted into and held by the distal end hard portion 31 in such a manner that the observation optical member 22 which is provided at the distal end of the imaging unit 32 and has a wide viewing angle is arranged at the distal end portion 10. The imaging unit 32 includes the observation optical member 22, an observation optical system 22a which is provided at a proximal end side of the observation optical member 22 and includes plural lenses, a cover glass 22b which is provided at a proximal end side of the observation optical member 22a, and an imaging element 22c which is a solid-state imaging sensor such as a CCD provided at a proximal end side of the cover glass 22b. The imaging unit 32 further includes a board (not shown) on which various types of circuits are formed and which is connected to the imaging element 22c. Still further, the board is connected to a signal cable (not shown). The signal cable penetrates through the insertion portion 3 and is connected to the video processor 6. The imaging unit 32 is fixed to the distal end hard portion 31 with a filling member or the like not shown.

In the embodiment, each of the illumination members 23 includes an illumination lens 33a which is an optical member for illumination and an optical fiber bundle 33b which is a light guide. The illumination member 23a includes an illumination lens 33aa and an optical fiber bundle 33ba which is a light guide. Though not shown, the illumination member 23b includes an illumination lens 33ab and an optical fiber bundle 33bb which is a light guide. The illumination member 23c includes an illumination lens 33ac and an optical fiber bundle 33bc which is a light guide. Hereinafter, the illumination lenses 33aa, 33ab, and 33ac may be collectively referred to as the illumination lens 33a. The optical fiber bundles 33ba, 33bb, and 33bc may be collectively referred to as the optical fiber bundle 33b.

A distal end portion of the optical fiber bundle 33b is fixed inside a metal pipe 33c, respectively, by bonding agent or the like. The distal end portion of the optical fiber bundle 33b and the illumination lens 33a are inserted and fixed inside a frame 33d. The illumination member 23 is secured to the distal end hard portion 31 with a setscrew. A part of the metal pipe 33c and the optical fiber bundle 33b are covered by an outer cladding tube 33e. The outer cladding tube 33e is wound round with a string 33f and thus fixed to the metal pipe 33c.

The proximal end portion of the distal end hard portion 31 is fixed to a portion of a bending distal end piece 35. The proximal end side of the distal end hard portion 31 and the bending distal end piece 35 are covered by an outer cladding tube 36. The outer cladding tube 36 is wound round with a string 37 and thus fixed to the metal pipe 33c.

The metal pipe 33c of each of the illumination members 23a and 23b is bent at a predetermined position P1 in the middle, and accordingly, the optical fiber bundles 33ba and 33bb are bent following the bent shape of the metal pipe 33c. Therefore, optical axes of the illumination lenses 33aa and 33ab which emit illumination light, or in other words, axes 33LA and 331B defined by centers of illumination ranges of the illumination members 23a and 23b, respectively, are not parallel with an optical axis 32LA which is an optical axis of the observation optical system 22a and also an optical axis of the observation optical member 22. More specifically, the optical axes 33LA and 33LB are inclined with respect to the optical axis 32LA by a predetermined angle θ1 as the optical axes 33LA and 33LB extend toward a distal end side from a tip point of the imaging unit 32 in an observation direction of the optical axis 32LA. The angle θ1 formed between each of the optical axes 33LA and 33LB and the optical axis 32LA is, for example, 8°.

In other words, the illumination lens 33aa of the illumination member 23a and the illumination lens 33ba of the illumination member 23b that form a first illumination member are each provided on an inclined portion 31c of the distal end surface 21. Specifically, since the observation optical member 22 has a wide viewing angle, for example 170° or more, optical axes (axes defined by centers of illumination areas) 33LA and 33LB of the two illumination lenses 33aa and 33ba form the angle θ1 with respect to the optical axis 32LA as described above on the distal end surface 21 of the distal end portion 10, so that a wide area of the observed area can be illuminated. Hence, a plane portion covering each of the planes perpendicular to the optical axes 33LA and 33LB, in other words, the inclined portion 31c of the distal end surface 21 is not parallel to a plane perpendicular to the optical axis 32LA, and rather is inclined by the angle θ1. Thus, the illumination window is provided on the inclined portion of the distal end surface 21 so that the optical axis of the illumination member is directed a radially outward direction of the insertion portion 3, so that the insertion portion 3 can be thinned down. In the embodiment, the angle formed by each of the optical axes 33LA and 33LB of the illumination members 23a and 23b and the optical axis 32LA is the same angle θ1. However, the two angles may be different from each other.

An optical axis 33LC of the illumination lens 33ac corresponding to the illumination member 23c which forms a second illumination member extends toward a distal end side in a direction substantially parallel to the optical axis 32LA. An angle formed by the optical axis 33LC and the optical axis 32LA is approximately 0°.

The illumination member 23c is arranged at a position inside the distal end portion 10 as is set based on an endoscopic image. For example, the illumination member 23c is arranged near the treatment instrument insertion channel, and in a lower direction of the endoscopic image (in other words, lower direction of the imaging unit 32 corresponding to a lower side of the endoscopic image). This is because there is generally a sufficient space around the treatment instrument insertion channel, and the illumination member 23c, i.e., the illumination lens 33ac and the optical fiber bundle 33bc can be arranged with a relatively high degree of freedom. When the illumination member 23c is arranged closer to the treatment instrument insertion channel than the illumination members 23a and 23b, the illumination light emitted from the illumination member 23c can surely illuminate the treatment instrument penetrating through the treatment instrument insertion channel and protruding from the distal end portion 10 and an area to be treated inside the body cavity (in particular, an area relatively close to the distal end portion 10).

Further, the illumination member 23c is not on the same plane as a plane on which the observation optical member 22 is placed at the distal end surface 21 of the distal end portion 10. In other words, the second illumination member 23c is located closer than the observation optical member 22 to the proximal end side in an axial direction of the insertion portion 3. More specifically, the distal end surface of the illumination lens 33a is arranged at the proximal end side a predetermined distance away from the lens surface of the observation optical member 22 in the direction of the optical axis 32LA of the observation optical member 22 so that light emitted from the illumination lens 33a does not come into the observation optical member 22.

The above arrangement is applied since the observation optical member 22 having a wide viewing angle tends to receive surrounding light easily. In particular, when the observation optical member 22 is a meniscus lens, some portion protrudes from the distal end surface 21, and such portion tends to receive surrounding light more easily. When the observation optical member 22 and the illumination lens 33c are on the same plane perpendicular to the optical axis 32LA at the distal end surface 21, the light emitted from the illumination lens 33ac comes into the observation optical member 22 and might cause flare on the observation image. Therefore, as shown in FIG. 3, the illumination lens 33c is arranged at a depressed portion at the proximal end side where the cover 31a has a step so that the illumination lens 33c is placed at the proximal end side a predetermined distance d1 away from the observation optical member 22. The cover 31a has a tapered surface at a portion between the observation optical member 22 and the illumination lens 33c, and the tapered surface forms a predetermined angle θ2 with respect to a plane of the illumination lens 33c. The predetermined angle θ2 is, for example, 8°.

The meniscus lens employed in the observation optical member 22 of the embodiment is advantageous in that the angle of view is relatively uniform regardless of fluctuation in parts and assembly in comparison with a flat lens.

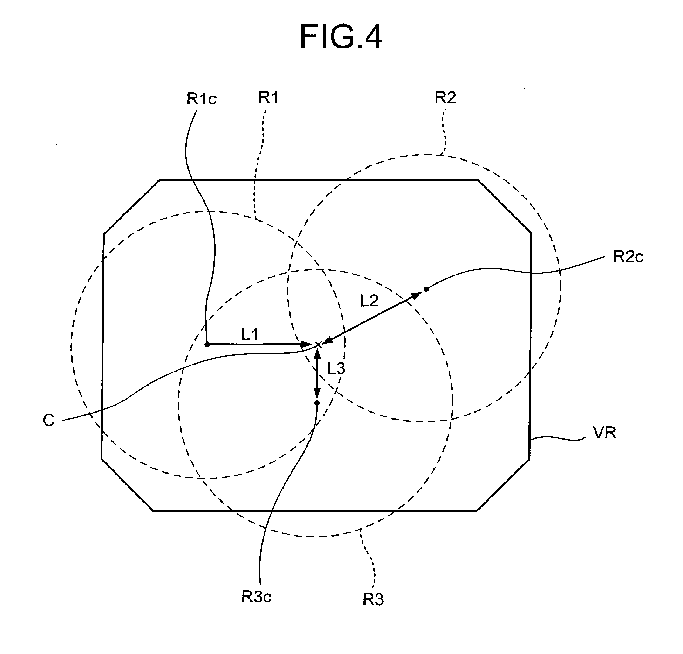

FIG. 4 is a diagram illustrating a relation among the observed area whose image is captured by the observation optical member 22, and an area illuminated by three illumination lenses 33a. In FIG. 4, an observed area VR which is an imaged area is substantially octagonal. Here, the observed area VR may be rectangular, circular, or the like. Further, in the embodiment, the imaging element 22c has a rectangular light receiving portion at which the imaging element 22c receives light from the observed area via the observation optical member 22, the observation optical system 22a, and the cover glass 22b. Among three illumination members 23, two illumination members 23a and 23c are arranged respectively close to one of two sides of the light receiving portion of the imaging element 22c, while the illumination member 23b is arranged close to a corner of the light receiving portion of the imaging element 22c. Here, the illumination member 23c is arranged at a position in the distal end portion 10 as is set based on an image captured by the imaging element 22c, in other words, the endoscopic image of the observed area VR. More specifically, the illumination member 23c is arranged in the distal end portion 10 at a position corresponding to a lower side of the endoscopic image of the observed area VR (in other words, a position close to the surroundings of the lower side to the imaging unit 32), for example. Therefore, even when there are only a few illumination members 23, the entire observed area can be well illuminated. Further, since the two illumination members 23 are arranged close to two side, respectively, close to the center of the light receiving portion of the imaging element 22c, the insertion portion 3 can be thinned down compared with that in an apparatus having two illumination members 23 arranged close to the corner.

The observed area VR which is an area to be observed, e.g., an inner wall of the body cavity, is illuminated by the illumination light from the three illumination members 23. As described above, in the embodiment, at least one illumination member 23c among three illumination members emits illumination light to an illumination range R3 along the optical axis 33LC. Hence, a central position R3c of the illumination range R3 is closer to a center C of the observed area VR than central positions R1c and R2c of illumination ranges R1 and R2 illuminated with the illumination light from the other two illumination lenses 33aa and 33ab. In other words, the direction of the optical axis 33LC is set so that distance L3 is shorter than distances L1 and L2, where L1, L2, and L3 represent distances from the center C of the observed area VR to the central positions R1c, R2c, and R3c, respectively.

Therefore, with the endoscope of the embodiment, a wide area of a desired observed area is illuminated while an area around the central portion of the observed area can be surely illuminated. Therefore, the illuminance distribution does not show decrease in illuminance at the central portion of the observed area even when the area is observed at a relatively short distance with an endoscope having a wide viewing angle, and preferably observability is obtained.

A modification of the embodiment will be described. In the embodiment described above, the optical axis 33LC of the illumination member 23c is substantially parallel to the optical axis 32LA of the observation optical member 22 with respect to the direction of extension of the optical axis toward the distal end. In the modification, the optical axis 33LC forms a predetermined angle θ3 with the optical axis 32LA of the observation optical member 22.

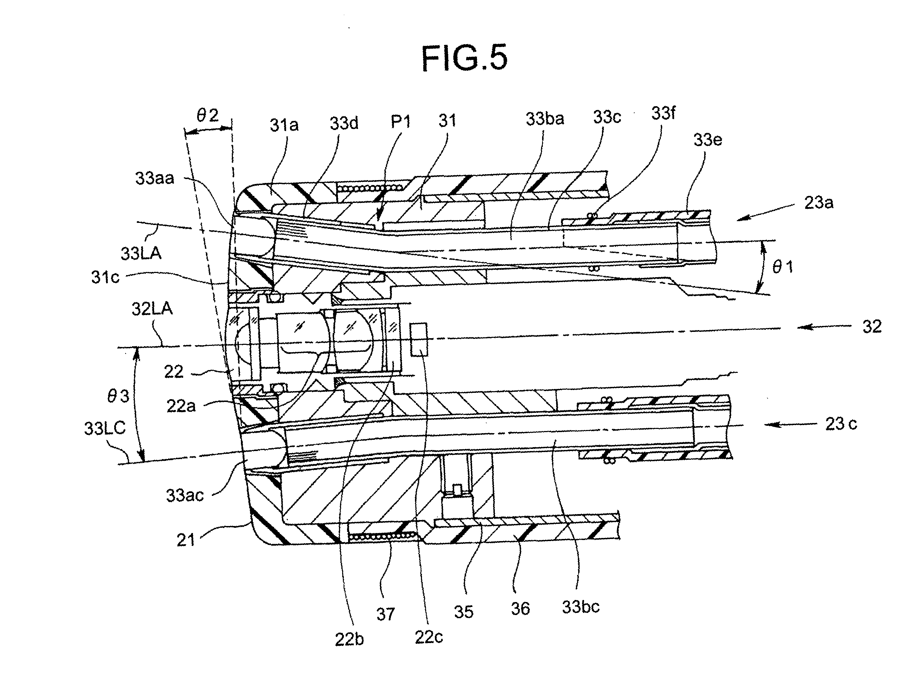

FIG. 5 is a sectional view of a distal end portion according to the modification of the embodiment.
FIG. 5 is a sectional view of the distal end portion 10 along line P-P of FIG. 2. The same elements as those of the embodiment shown in FIGS. 1 to 4 are denoted by the same reference characters and the description thereof will not be repeated. As shown in FIG. 5, the optical axis 33LC of the illumination member 23c forms the predetermined angle θ3 with the optical axis 32LA with respect to the direction of extension of the optical axis toward the distal end. The optical axes 33LA and 33LB of the other two illumination members 23a and 23b each form the angle θ1 mentioned above with the optical axis 32LA. The angle θ3 is smaller than the angle θ1, and is for example 3°, and similarly to the embodiment described above, the central position R3c of the illumination range R3 is set to be closer to the center C of the observed area VR than the central positions R1c and R2c of the illumination ranges R1 and R2 illuminated with the illumination light from the other two illumination members 23a and 23b. Therefore, the angle θ3 is set so that the distance L3 is shorter than each of the distances L1 and L2, where L1, L2, and L3 represent the distances from the center C of the observed area VR to the respective central positions R1c, R2c, and R3c.

Therefore, also in the modification, the illuminance distribution does not show decrease in illuminance at the central portion of the observed area VR, in other words, there is no deficiency in light intensity at the central position even when the operator tries to observe the observed area at a relatively short distance, and therefore favorable observability can be obtained.

As described above, according to the embodiment of the present invention and the modification thereof, a wide area of a desired observed area is illuminated while the portion around the central portion of the observed area can be surely illuminated, whereby the illuminance distribution does not show decrease in illuminance at the central portion of the observed area even when the area is observed at a relatively short distance with the use of an endoscope having a wide viewing angle, and the endoscope with favorable observability can be obtained.

In the embodiment and the modification as described above, the illumination members 23a, 23b, and 23c include the illumination lenses 33aa, 33ab, and 33ac, and the corresponding optical fiber bundles 33ba, 33bb, and 33bc, respectively. The present invention is not limited thereto, and an illuminating unit may be implemented with an LED or the like and provided near the proximal end side of each of the illumination lenses 33aa, 33ab, and 33ac, in place of the illumination members 23a, 23b, and 23c, and the insertion portion 3 may be provided with a power supply line to supply power to the illuminating unit. The power supply line includes a signal line to control the illumination by the illuminating unit. In other words, it is sufficient if the illuminating light is emitted from each position of the illumination lenses 33aa, 33ab, and 33ac in directions of the optical axis 33LA, 33LB, and 33LC, respectively. An illumination member including the illumination lens 33aa, 33ab, and 33ac and the illuminating unit may be arranged near inside the openings for the illumination members 23a, 23b, and 23c formed on the distal end surface 21 of the distal end portion 10 described above.

In the embodiment and the modification as described above, the three illumination members 23 are arranged near either the side or the corner of the light receiving portion of the imaging element 22c. The present invention is not limited thereto. The imaging element 22c itself may have a casing-like shape which has a rectangular surface, and the three illumination members 23 may be arranged near either the side or the corner of the rectangular surface.

Further, in the embodiment and the modification as described above, the illumination member 23c in which the distance between the center C of the observed area VR and the central position of the illumination range is shortest is arranged at a position corresponding to the lower side of the endoscopic image of the observed area VR in the distal end portion 10. The present invention is not limited thereto. As far as the distance L3 between the central position R3c of the illumination range R3 and the center C is shorter than each of the distances L1 and L2 between the central positions R1c and R2c of the illumination ranges R1 and R2 and the center C, the illumination member 23c may be placed at a position corresponding to an upper side, a right side, or a left side of the endoscopic image of the observed area VR in the distal end portion 10. In brief, it is sufficient if the illumination member 23c is arranged so that the central position R3c of the illumination range R3 is formed near the center C of the observed area VR, and the illumination members 23a and 23b are arranged based on the position of the illumination member 23c so that the distance L3 is shorter than each of the distances L1 and L2.

### INDUSTRIAL APPLICABILITY

As can be seen from the foregoing, the endoscope according to the present invention is useful for capturing an observation image with a wide viewing angle and for observing an interior of a body cavity, and more particularly, suitable for illuminating a wide area of an observed area inside the body cavity and allowing a secure illumination of an observed area at a relatively short distance from a distal end of the insertion portion.

## Claims

1. An endoscope having an insertion portion to be inserted into a subject body, comprising:
an observation optical member which is provided in a distal end portion of the insertion portion and serves for an observation of the subject body;
at least two first illumination members each of which is arranged around the observation optical member and having an axis defined by a center of an illumination range forming a predetermined angle with an optical axis of the observation optical member in a radially outward direction of the insertion portion; and
a second illumination member which is arranged around the observation optical member,
wherein an angle formed by an axis defined by a center of an illumination range of the second illumination member and the optical axis of the observation optical member is smaller than the predetermined angle formed by the axis defined by the center of the illumination range of the first illumination member and the optical axis of the observation optical member.

2. The endoscope according to claim 1, wherein the angle formed by the axis defined by the center of the illumination range of the second illumination member and the optical axis of the observation optical member is substantially 0°.

3. The endoscope according to claim 2, wherein the second illumination member is placed at a position closer to a proximal end side than the observation optical member in an axial direction of the insertion portion.

4. The endoscope according to any one of claims 1 to 3, further comprising
an imaging unit which receives light from an observed area in the subject body via the observation optical member to capture an image of the observed area, wherein
the second illumination member is arranged at a position, which is set based on an image of the observed area, in the distal end portion.

5. The endoscope according to any one of claims 1 to 3,
wherein a number of the first illumination members is two.

6. A distal end structure of an endoscope having an insertion portion to be inserted inside a subject body, comprising:
an observation optical member which is provided in a distal end portion of the insertion portion and serves for an observation of the subject body;
at least two first illumination members each of which is arranged around the observation optical member and having an axis defined by a center of an illumination range forming a predetermined angle with an optical axis of the observation optical member in a radially outward direction of the insertion portion; and
a second illumination member which is arranged around the observation optical member,
wherein an angle formed by an axis defined by a center of an illumination range of the second illumination member and the optical axis of the observation optical member is smaller than the predetermined angle formed by the axis defined by the center of the illumination range of the first illumination member and the optical axis of the observation optical member.

7. An endoscope having an insertion portion to be inserted into a subject body, comprising:
an observation optical member which is provided in a distal end portion of the insertion portion and serves for an observation of the subject body;
first illumination members each of which is provided in the distal end portion of the insertion portion, having an axis defined by a center of an illumination range forming a predetermined angle with an optical axis of the observation optical member in a radially outward direction of the insertion portion; and
a second illumination member which is provided in the distal end portion of the insertion portion,
wherein an angle formed by an axis defined by a center of an illumination range of the second illumination member and the optical axis of the observation optical member is smaller than the predetermined angle formed by the axis defined by the center of the illumination range of the first illumination member and the optical axis of the observation optical member.
